# EUROPÄISCHE PATENTSCHRIFT

(11) **EP 1 092 155 B1**
(45) Veröffentlichungstag und Bekanntmachung des Hinweises auf die Patenterteilung: **02.01.2003**
(21) Anmeldenummer: 99945931.6
(22) Anmeldetag: 01.07.1999
(51) Int. Cl.: G01N 33/574

(54) **VERFAHREN ZUR FRÜHEN DIAGNOSE VON CARCINOMEN**
METHOD FOR EARLY DIAGNOSIS OF CARCINOMAS
METHODE DE DIAGNOSTIC PRECOCE DE CARCINOMES

(30) Priorität: 01.07.1998 DE 19829473
(43) Veröffentlichungstag der Anmeldung: 18.04.2001
(73) Patentinhaber: Von Knebel Doeberitz, Magnus, 69120 Heidelberg (DE); Deutsches Krebsforschungszentrum Stiftung des öffentlichen Rechts, 69120 Heidelberg (DE)
(72) Erfinder: VON KNEBEL DOEBERITZ, Magnus, Chir.Univ.klinik, Im Neuenheimer Fld 110, 69120 Heidelberg (DE); SPITKOVSKY, Dimitri, D-50931 Köln (DE)
(74) Vertreter: Schüssler, Andrea, Dr.
(86) Internationale Anmeldenummer: DE9902094
(87) Internationale Veröffentlichungsnummer: WO00001845

(56) Entgegenhaltungen:
- WO-A-94/17414
- CHEMICAL ABSTRACTS, vol. 126, no. 10, 10. März 1997 (1997-03-10) Columbus, Ohio, US; abstract no. 129966, XP002125853 & T. ZONG ET AL.: "Concurrent overexpression of cyclin D1 and cyclin-dependent kinase 4 ( Cdk4 ) in intestinal adenomas from multiple intestinal neoplasia (Min) mice and human familial adenomatous polyposis patients" CANCER RESEARCH, Bd. 57, Nr. 1, 1997, Seiten 169-175, Nashvile TN USA
- CHEMICAL ABSTRACTS, vol. 124, no. 17, 22. April 1996 (1996-04-22) Columbus, Ohio, US; abstract no. 228401, XP002125854 & D.C. BETTICHER ET AL.: "Prognostic significance of CCND1 (cyclin D1) overexpression in primary resected non-small-cell lung cancer" BRITISH JOURNAL OF CANCER, Bd. 73, Nr. 3, 1996, Seiten 294-300, Cambridge UK
- V. ESPOSITO ET AL.: "Prognostic role of the cyclin-dependent kinase inhibitor p27 in non-small cell lung cancer " CANCER RESEARCH, Bd. 57, 15. August 1997 (1997-08-15), Seiten 3381-3386, XP002912136 Baltimore MD USA

## Beschreibung

Die vorliegende Erfindung betrifft ein Verfahren zur frühen Diagnose von Carcinomen sowie ihren Vorstufen, insbesondere von Carcinomen des oberen Respirationstraktes oder des Anogenitaltraktes.

Seit Mitte der 50iger Jahre werden Vorsorgeprogramme für die verschiedensten Carcinome angeboten. Für das Cervix-Carcinom basieren diese hauptsächlich auf der morphologischcytologischen Untersuchung von Zellabstrichen der Cervix-Uteri, sog. Pap-Test, die in regelmäßigen Abständigen bei der Frau ab dem 20. Lebensjahr im Rahmen von gynäkologischen Routineuntersuchungen entnommen werden. Anhand der Morphologie der Zellen werden die Abstriche in unterschiedliche Ausprägungsgrade dysplastischer Zellveränderungen eingeteilt. Die-se Ausprägungsgrade werden mit normal, milde Dysplasie, mittlere Dysplasie, schwere Dysplasie bzw. invasives Carcinom entsprechend Pap I-V bezeichnet. Bei einem auffälligen Ergebnis des Pap-Tests wird eine kleine Biopsie entnommen und einer histopathologischen Untersuchung unterworfen, durch welche die Art und Ausprägung der Dysplasie festgestellt und als cervikale intraepitheliale Neoplasie (CINI-III) eingestuft werden.

Trotz aller Vorsorgeprogramme ist das Cervix-Carcinom mit über 400000 Neuerkrankungen pro Jahr das zweithäufigste Carcinom der Prau. Dies liegt u.a. daran, daß die Ergebnisse des Pap-Tests bis zu 30 % falsch-negativ sind.

Aus WO 94/17414 ist bekannt, daß die Zykline A, B und E sowie die Zyklin-abhängige Kinase CDC2 in Brustkrebszellen überexprimiert werden.

Ferner ist aus Cancer Research, Bd. 57, Nr. 1 (1997), 169-175 bekannt, daß in intestinalen Adenomen von FAP-Patienten eine Überexpression von Zyklin D1 und Zyklin-abhängiger Kinase 4 (CdK4) vorliegt.

Der vorliegenden Erfindung liegt die Aufgabe zugrunde, ein Verfahren bereitzustellen, mit dem das Cervix-Carcinom frühzeitig und zuverlässig diagnostiziert werden kann. Ferner sollten durch das Verfahren gutartige entzündliche oder metaplastische Veränderungen von dysplastischen Präneoplasien abgegrenzt werden können.

Erfindungsgemäß wird dies durch die Gegenstände in den Patentansprüchen erreicht.

Die vorliegende Erfindung beruht auf den Erkenntnissen des Anmelders, daß Zellzyklus-Regulatorproteine in vielen Carcinomen, z.B. solchen des oberen Respirationstraktes oder Anogenital-Carcinomen, insbesondere Cervix-Carcinom, bzw. Vorstufen dieser Carcinome, überexprimiert sind. Mit Zellzyklus-Regulatorproteinen sind Zyklin-abhängige Kinase-Inhibitoren gemeint. Diese regulieren Zyklin-abhängige Kinasen. Beispiele von Zyklin-abhängigen Kinase-Inhibitoren sind die Proteine p14, p15, p16, p19, p21 und p27. Der Anmelder hat gefunden, daß die Stärke der Überexpression der Zellzyklus-Regulatorproteine mit dem Grad der Zell-Dysplasie korreliert.

Erfindungsgemäß werden die Erkenntnisse des Anmelders für ein Verfahren zur frühen Diagnose von Carcinomen und ihren Vorstufen genutzt, das die Bestimmung der Überexpression eines Zellzyklus-Regulatorproteins in einer Körperprobe umfaßt, wobei das Zellzyklus-Regulatorprotein ein Zyklin-abhängiger Kinase-Inhibitor ist.

Der Ausdruck "Carcinome und ihre Vorstufen" umfaßt Carcinome jeglicher Art und Herkunft bzw. Vorstufen dieser. Beispielsweise können es Carcinome des oberen Respirationstraktes oder Anogenital-Carcinome, insbesondere das Cervix-Carcinom, sein. Von letzterem sind auch seine Vorstufen, z.B. cervikale intraepitheliale Neoplasie (CINI-III), Carcinoma in situ (CIS), etc. besonders zu nennen.

Der Ausdruck "Zellzyklus-Regulatorprotein" betrifft Zyklin-abhängige Kinase-Inhibitoren. Beispiele der Zyklin-abhängigen Kinase-Inhibitoren sind die Proteine p14, p15, p16, p18, p19, p21 und p27, wobei p16 bevorzugt ist.

Der Ausdruck "Körperprobe" umfaßt jegliche Körperproben, in denen Zellzyklus-Regulatorproteine nachgewiesen werden können. Beispiele solcher Körperproben sind Blut, Abstriche, Sputum, Urin, Stuhl, Liquor, Galle, gastrointestinale Sekrete, Lymphflüssigkeit, Knochenmark, Organpunktate und Biopsien. Insbesondere sind Abstriche und Biospien angesagt, wenn es sich um den Nachweis von Anogenital-Carcinomen, z. B. Cervix-Carcinom, handelt.

Der Ausdruck "Bestimmung der Überexpression von Zellzyklus-Regulatorproteinen" umfaßt jegliche Verfahren, die sich zum Nachweis der Expression von Zellzyklus-Regulatorproteinen oder ihrer kodierenden mRHAs bzw. einer Amplifikation der entsprechenden Gene eignen. Zur Feststellung einer Überexpression bietet es sich dann an, die zu untersuchende Körperprobe mit einer entsprechenden Körperprobe zu vergleichen, die aus gesunden Personen stammt. Eine solche kann in standardisierter Form vorliegen. Der Nachweis der (Über)Expression von Zellzyklus-Regulationsproteinen kann auf Nukleinsäure- bzw. Protein-Ebene erfolgen. Für den Nachweis auf Protein-Ebene können z.B. Antikörper verwendet werden, die gegen Zellzyklus-Regulatorproteine gerichtet sind. Diese Antikörper können in den verschiedensten Verfahren wie, Western-Blot, ELISA oder Immunpräzipitation eingesetzt werden. Günstig kann es sein, wenn die Antikörper auf festen Trägern, wie Teststreifen oder Latex-Partikel, fixiert sind.

Mit der vorliegenden Erfindung ist es möglich, Carcinome frühzeitig, d.h. in ihren Vorstufen, zu diagnostizieren.

Ein weiterer Gegenstand der vorliegenden Erfindung ist ein Kit zur Durchführung eines erfindungsgemäßen Verfahrens. Ein solcher Kit umfaßt:
(a) ein Reagenz zum Nachweis der Expression eines Zellzyklus-Regulatorproteins, z.B. einen gegen ein solches gerichteten Antikörper oder eine für ein solches kodierende Nukleinsäure bzw. Teile davon,
(b) übliche Hilfsmittel, wie Puffer, Träger, Marker, etc., und ggfs.
(c) ein Mittel für Kontrollreaktionen, z.B. ein Zellzyklus-Regulatorprotein, eine für ein solches kodierende Nukleinsäure bzw. Teile davon, oder eine Zellpräparation z.B. einen Gewebeschnitt oder auf einem Objektkörper fixierte Zellen.

Für die einzelnen Komponenten des Kits gelten vorstehende Ausführungen entsprechend. Ferner können von den einzelnen Komponenten ein oder mehrere Vertreter vorliegen.

Mit der vorliegenden Erfindung ist es möglich, Carcinome frühzeitig zu diagnostizieren. Insbesondere können Vorstufen von Carcinomen frühzeitig erkannt werden. Zu betonen ist auch, daß gutartige entzündliche oder metaplastische Veränderungen von dysplastischen Präneoplasien abgegrenzt werden können. Kennzeichnend ist ferner, daß die durch ein erfindungsgemäßes Verfahren erzielten Ergebnisse nicht einer subjektiven Bewertung unterliegen, wodurch z.B. die falsch-negativen bzw. falsch-positiven Ergebnisse eines Pap-Tests oder von histologischen Präparationen vermieden werden können. Desweiteren zeichnet sich die vorliegende Erfindung durch schnelle und einfache Handhabung aus, wodurch sie für große Screening-Maßnahmen, insbesondere auch in Ländern der Dritten Welt, geeignet ist. Somit stellt die vorliegende Erfindung einen wichtigen Beitrag zur modernen Diagnostik von Krebserkrankungen dar.

### Kurze Beschreibung der Zeichnung.

- **Fig. 1**: zeigt den Nachweis der Überexpression von cdk4 (als Vergleich) in HPV16-transformierten cervicalen Carcinoma-Zellen CaSki. Die Angaben 4 h, 8 h, 12 h, 24 h weisen auf die Zeitpunkte der Entnahme der Zellextrakte hin. Die Angabe co bedeutet Kontrolle während arr die Zugabe des Serums andeutet.
- **Fig. 2**: zeigt den Nachweis der Überexpression von cdk6 (als Vergleich) und p19 in HPV16-transformierten NIH3T3-Zellen. Die Angabe co bedeutet Kontrolle.

Die Erfindung wird durch die folgenden Beispiele erläutert.

### Beispiel 1: Nachweis der Überexpression von p16 in Biopsien der Cervix Uteri

(A) Aus 20 Biopsien der Cervix Uteri, die alle Grade der dysplastischen Progression von Normalgewebe (n=2) über CIN I (n=4), II (n=4), III (n=5) Läsionen bis hin zum invasiven Carcinom (n=5) umfassen, werden Paraffin-Schnitte mit einer Dicke von 3-5µm hergestellt. Diese werden 2 x 10 min in Xylol entparaffiniert und mit Ethanol rehydriert. Die Demaskierung der Antigene erfolgt in 10 mM Citrat-Puffer (pH 6,0) im Autoklaven bei 110°C für 10 min. Anschließend werden die endogenen Peroxidasen mit 0,25 % H₂O₂ in PBS inaktiviert. Nach Blockierung unspezifischer Bindungstellen mit Pferde-Serum (Vectastain ABC detection kit, Vector Laboratories, Burlingame, Kalifornien, USA) für 20 min bei Raumtemperatur, werden die Schnitte für 45 min bei Raumtemperatur mit einem p16-spezifischen, monoklonalen Antikörper (Neomarkers, Fremont, Kalifornien, USA) in Anwesenheit von 3 % fötalem Kälberserum inkubiert. Zum Nachweis der p16-Antikörper-Bindung wird dann ein biotinylierter Sekundär-Antikörper (Pferd-Anti-Maus-IgG, Vectastain kit, vgl. vorstehend) für 30 min hinzugegeben. Anschließend erfolgt der Nachweis des gebundenen Sekundär-Antikörpers mit den Reagenzien und nach Anweisung des Vectastain kits und eine Kern-Gegenfärbung mit Meyerscher Hämalaunlösung. Es zeigt sich, daß in Zellen mit Dysplasien eine Überexpression von p16 vorliegt. Ferner zeigt sich, daß die Stärke der Überexpression von p16 mit dem Grad der Zell-Dysplasie korreliert.
(B) Ferner werden aus 78 Biopsien der Cervix Uteri Paraffin-Schnitte hergestellt. Die Biopsien betreffen Normalgewebe (n = 12), dysplastische Läsionen der Stufen CIN I (n = 15), II (n = 14) und III (n = 18) sowie invasive Carcinome (n = 19). Die Paraffin-Schnitte werden, wie in (A) beschrieben, behandelt. Es werden die in Tabelle 1 angegebenen Daten erhalten.

**Tabelle 1**

| | p16 Expressionsstärke | | | | |
|---|---|---|---|---|---|
| Histologie | n= | - | + | ++ | +++ |
| normal | 12 | 9 | 3 | | |
| CIN I | 15 | 10 | 3 | 2 | |
| CIN **II** | 14 | 1 | 4 | 9 | |
| CIN III | 18 | | | 9 | 9 |
| CxCa | 19 | | | 1 | 18 |
| insgesamt | 78 | 20 | 10 | 21 | 27 |

Aus den Daten von Tabelle 1 geht hervor, daß p16 in Zellen von Dysplasien und invasiven Carcinomen überexprimiert wird, wobei die Überexpression mit dem Grad der Dysplasie hin zum invasiven Carcinom zunimmt.
(C) Desweiteren werden Paraffin-Schnitte von 180 Biopsien der Cervix Uteri, wie in (A) beschrieben, behandelt. Ferner wird die prozentuale Zellzahl bestimmt, die mit dem vorstehenden p16-spezifischen monoklonalen Antikörper reagiert. Desweiteren wird zwischen HPV positiven und HPV negativen Dysplasien bzw. invasiven Carcinomen unterschieden. Es werden die in Tabelle 2 angegebenen Daten erhalten.

**Tabelle 2**

| **Prozentsatz von Zellen, die p16 überexprimieren** | | |
|---|---|---|
| | n | gemittelter Prozentsatz ± Standardabweichung |
| CIN I | 32 | 54.9 ± 24.0 |
| HPV negativ | 17 | 54.0 ± 27.2 |
| HPV positiv | 15 | 55.9 ± 21.0 |
| | | |
| CIN II | 32 | 70.8 ± 18.9 |
| HPV negativ | 14 | 76.0 ± 15.8 |
| HPV positiv | 18 | 66.8 ± 20.5 |
| | | |
| CIN III | 60 | 92.4 ± 10.2 |
| HPV negativ | 9 | 94.4 ± 7.5 |
| HPV positiv | 51 | 92.1 ± 10.7 |
| | | |
| Invasives Carcinom | 58 | 97.8 ± 5.2 |
| HPV negativ | 5 | 96.4 ± 8.1 |
| HPV positiv | 53 | 97.9 ± 4.9 |

Aus den Daten der Tabelle 2 geht hervor, daß p16 sowohl in HPV positiven als auch HPV negativen Zellen von Dysplasien und invasiven Carcinomen überexprimiert wird. Kontrollen mit Normalgewebe bestätigen dies. Ferner zeigen die Daten, daß der Prozentsatz an Zellen, die mit p16 reagieren, mit dem Grad der Dysplasie hin zum invasiven Carcinom zunimmt.

### Beispiel 2: Nachweis der Überexpression von Zellzyklus-Regulatorproteinen in HPV-transformierten Zellen

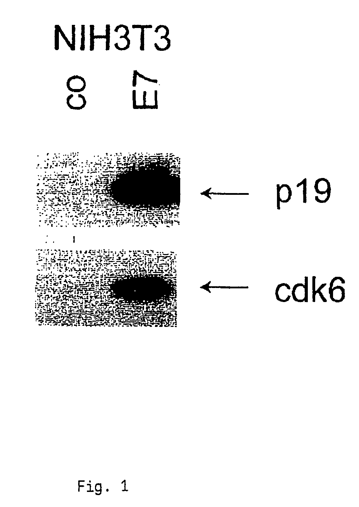

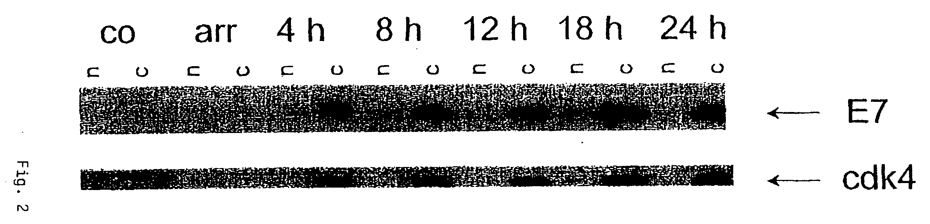

(A) Cervicale Carcinoma-Zellen CaSki, die mit HPV16 transformiert sind, werden in Abwesenheit von Serum 72 h kultiviert. Nach Zugabe von Serum werden zu verschiedenen Zeitpunkten Zellextrakte gewonnen, einer SDS-PAGE unterworfen und auf PVDF-Membranen (Du Pont) transferiert. Die Expression von cdk4 (als Vergleich) wird mit polyklonalem Antiserum (1 : 1000) von Santa Cruz bestimmt. Ferner wird die Expression von HPV16 - E7 Protein mit einem monoklonalen Antikörper gegen HPV16 E7 (1 : 50) von Triton bestimmt. Der Nachweis der einzelnen Immunreaktionen erfolgt über Peroxidase-gekoppelte Zweit-Antikörper und ein Chemielumineszenz-Nachweissystem (NEN, Du Pont).
   Es zeigt sich, daß cdk4 überexprimiert wird (vgl. Fig. 1).
(B) NIH3T3-Zellen werden mit HPV16 transformiert, wodurch eine Expression von HPV16-E7 Protein erhalten wird. Zellextrakte der transformierten Zellen werden, wie in (A) beschrieben, gewonnen und behandelt. Zum Nachweis der Expression von cdk6 (als Vergleich) bzw. p19 werden polyklonale Antiseren (1 : 1000) von Santa Cruz eingesetzt. Hinsichtlich des Nachweises der Expression von HPV16-E7 Protein und des Nachweises der einzelnen Immunreaktionen wird auf vorstehende Ausführungen unter (A) verwiesen.
   Es zeigt sich, daß cdk6 (als Vergleich) und P19 überexprimiert werden (vgl. Fig. 2).

## Patentansprüche

1. Verfahren zur frühen Diagnose von Carcinomen und ihren Vorstufen, umfassend die Bestimmung der Überexpression eines Zellzyklus-Regulatorproteins in einer Körperprobe, wobei das Zellzyklus-Regulatorprotein ein Zyklin-abhängiger Kinase-Inhibitor ist.

2. Verfahren nach Anspruch 1, wobei die Carcinome solche des oberen Respirationstraktes sind.

3. Verfahren nach Anspruch 1, wobei die Carcinome Anogenital-Carcinome sind.

4. Verfahren nach Anspruch 3, wobei das Anogenital-Carcinom ein Cervix-Carcinom ist.

5. Verfahren nach einem der Ansprüche 1 - 4, wobei der Zyklin-abhängige Kinase-Inhibitor ein Protein p14, p15, p16, p18, p19, p21 oder p27 ist.

6. Verfahren nach einem der Ansprüche 1 - 5, wobei die Körperprobe Blut, Abstriche, Sputum, Urin, Stuhl, Liquor, Galle, Knochenmark, gastrointestinale Sekrete, Organpunktate bzw. Biopsin und/oder Lymphflüssigkeit ist.

7. Verfahren nach einem der Ansprüche 1 - 6, wobei die Bestimmung der Überexpression auf dem Nukleinsäure-Level erfolgt.

8. Verfahren nach einem der Ansprüche 1 - 6, wobei die Bestimmung der Überexpression auf dem Protein-Level erfolgt.

9. Verfahren nach Anspruch 8, wobei die Bestimmung durch einen gegen ein Zellzyklus-Regulatorprotein gerichteten Antikörper erfolgt.

10. Kit zur Durchführung des Verfahrens nach einem der Ansprüche 1 - 9, umfassend:
(a) ein Nachweisreagenz für die Expression eines Zellzyklus-Regulatorproteins, wobei das Zellzyklus-Regulatorprotein ein Zyklin-abhängiger Kinase-Inhibitor ist.
(b) übliche Hilfsmittel, wie Puffer, Träger, Marker, und ggfs.
(c) ein Mittel für Kontrollreaktionen.

11. Kit nach Anspruch 10, wobei das Reagenz ein gegen ein Zellzyklus-Regulatorprotein gerichteter Antikörper ist, wobei das Zellzyklus-Regulatorprotein ein Zyklin-abhängiger Kinase-Inhibitor ist.

12. Kit Nach Anspruch 10, wobei das Reagenz eine für ein Zellzyklus-Regulatorprotein kodierende Nukleinsäure oder Teile davon ist, wobei das Zellzyklus-Regulatorprotein ein Zyklin-abhängiger Kinase-Inhibitor ist.

13. Kit nach einem der Ansprüche 10-12, wobei das Mittel ein Zellzyklus-Regulatorprotein oder eine für ein solches kodierende Nukleinsäure oder Teile davon ist, wobei das Zellzyklus-Regulatorprotein ein Zyklin-abhängiger Kinase-Inhibitor ist.

14. Kit nach einem der Ansprüche 10-12, wobei das Mittel eine Zellpräparation oder auf einem Objektträger fixierte Zellen ist.

## Claims

1. A method for the early diagnosis of carcinomas and their preliminary stages, comprising determining the overexpression of a cell cycle regulatory protein in a body sample, wherein the cell cycle regulatory protein is a cyclin-dependent kinase inhibitor.

2. The method according to claim 1, wherein the carcinomas are those of the upper respiratory tract.

3. The method according to claim 1, wherein the carcinomas are anogenital carcinomas.

4. The method according to claim 3, wherein the anogenital carcinoma is a cervical carcinoma.

5. The method according to any of claims 1 to 4, wherein the cyclin-dependent kinase inhibitor is a protein p14, p15, p16, p18, p19, p21 or p27.

6. The method according to any of claims 1 to 5, wherein the body sample is blood, smears, sputum, urine, stool, liquor, bile, bone marrow, gastrointestinal secretions, organ punctates or biopsies and/or lymph.

7. The method according to any of claims 1 to 6, wherein the overexpression is determined via the nucleic acid level.

8. The method according to any of claims 1 to 6, wherein the overexpression is determined via the protein level.

9. The method according to claim 8, wherein an antibody directed against a cell cycle regulatory protein is employed for the determination.

10. A kit for carrying out the method according to any of claims 1 to 9, comprising:
(a) an analytical reagent for the expression of a cell cycle regulatory protein, the cell cycle regulatory protein being a cyclin-dependent kinase inhibitor,
(b) conventional auxiliary agents, such as buffers, carriers, markers, and optionally
(c) an agent for control reactions.

11. The kit according to claim 10, wherein the reagent is an antibody directed against a cell cycle regulatory protein, the cell cycle regulatory protein being a cyclin-dependent kinase inhibitor.

12. The kit according to claim 10, wherein the reagent is a nucleic acid coding for a cell cycle regulatory protein or fragments thereof, the cell cycle regulatory protein being a cyclin-dependent kinase inhibitor.

13. The kit according to any of claims 10 to 12, wherein the agent is a cell cycle regulatory protein or a nucleic acid coding for it or fragments thereof, the cell cycle regulatory protein being a cyclin-dependent kinase inhibitor.

14. The kit according to any of claims 10 to 12, wherein the agent is a cell preparation or cells fixed on a slide.

## Revendications

1. Procédé de diagnostic précoce de carcinomes et de leurs premiers stades, comprenant l'évaluation de la surexpression d'une protéine régulatrice du cycle cellulaire dans un échantillon corporel, la protéine régulatrice du cycle cellulaire étant un inhibiteur de la kinase cycline-dépendante.

2. Procédé selon la revendication 1 dans lequel les carcinomes sont des carcinomes des voies respiratoires supérieures.

3. Procédé selon la revendication 1 dans lequel les carcinomes sont des carcinomes ano-génitaux.

4. Procédé selon la revendication 3 dans lequel le carcinome ano-génital est un carcinome du col de l'utérus.

5. Procédé selon l'une des revendications 1 à 4 dans lequel l'inhibiteur de la kinase cycline-dépendante est une protéine p14, p15, p16, p18, p19, p21 ou p27.

6. Procédé selon l'une des revendications 1 à 5 dans lequel l'échantillon corporel est du sang, un frottis, une expectoration, de l'urine, une selle, du liquide céphalo-rachidien, de la bile, de la moelle osseuse, des sécrétions gastro-intestinales, des ponctions d'organe ou biopsies et/ou de la lymphe.

7. Procédé selon l'une des revendications 1 à 6 dans lequel l'évaluation de la surexpression a lieu au niveau des acides nucléiques.

8. Procédé selon l'une des revendications 1 à 6 dans lequel l'évaluation de la surexpression a lieu au niveau protéique.

9. Procédé selon la revendication 8 dans lequel l'évaluation a lieu à l'aide d'un anticorps dirigé contre une protéine régulatrice du cycle cellulaire.

10. Kit pour la réalisation du procédé selon l'une des revendications 1 à 9 comprenant :
(a) un réactif de détection pour l'expression d'une protéine régulatrice du cycle cellulaire, la protéine régulatrice du cycle cellulaire étant un inhibiteur de la kinase cycline-dépendante.
(b) des accessoires habituels comme un tampon, un support, un marqueur et, le cas échéant,
(c) un moyen pour des réactions de contrôle.

11. Kit selon la revendication 10 dans lequel le réactif est un anticorps dirigé contre une protéine régulatrice du cycle cellulaire, la protéine régulatrice du cycle cellulaire étant un inhibiteur de la kinase cycline-dépendante.

12. Kit selon la revendication 10 dans lequel le réactif est un acide nucléique codant la protéine régulatrice du cycle cellulaire ou une partie de cet acide nucléique, la protéine régulatrice du cycle cellulaire étant un inhibiteur de la kinase cycline-dépendante.

13. Kit selon l'une des revendications 10 à 12 dans lequel le moyen est une protéine régulatrice du cycle cellulaire ou un acide nucléique codant cette protéine ou une partie de cet acide nucléique, la protéine régulatrice du cycle cellulaire étant un inhibiteur de la kinase cycline-dépendante.

14. Kit selon l'une des revendications 10 à 12 dans laquelle le moyen est une préparation cellulaire ou des cellules fixées sur un porte-objet.
